# EUROPÄISCHE PATENTSCHRIFT

(11) **EP 1 193 520 B1**
(45) Veröffentlichungstag und Bekanntmachung des Hinweises auf die Patenterteilung: **18.01.2006**
(21) Anmeldenummer: 01120854.3
(22) Anmeldetag: 30.08.2001
(51) Int. Cl.: G02B 21/00

(54) **Operationsmikroskop**
Surgical microscope
Microscope chirurgical

(30) Priorität: 30.09.2000 DE 10048546
(43) Veröffentlichungstag der Anmeldung: 03.04.2002
(73) Patentinhaber: Carl Zeiss Surgical GmbH, 73447 Oberkochen (DE); Carl-Zeiss-Stiftung, trading as Carl Zeiss, 89518 Heidenheim (Brenz) (DE)
(72) Erfinder: Nägele, Ulrich, 73566 Bartholomä (DE); Pelzer, Martin, 89551 Königsbronn (DE); Duschek, Christian, 73575 Horn (DE); Gold, Ulrich, 73433 Aalen (DE); Düver, Erik, 73447 Oberkochen (DE); Quendt, Dieter, 73457 Essingen (DE)
(74) Vertreter: Gauss, Nikolai

(56) Entgegenhaltungen:
- US-A- 5 513 005
- US-A- 5 579 772
- US-A- 5 662 111
- US-B1- 6 434 416

## Beschreibung

Die Erfindung betrifft ein Operationsmikroskops nach dem Oberbegriff von Anspruch 1.

Ein derartiges Operationsmikroskop ist aus der JP 2000139949 bekannt, bei dem mittels zwei als CCD-Kameras ausgebildete Bildsensoren mit einem durch das Mikroskop-Hauptobjektiv durchtretenden Abbildungsstrahlengang ein auf einem chirurgischen Instrument angebrachte Markierung aus unterschiedlichen Perspektiven erfasst werden kann. Die den unterschiedlichen Perspektiven entsprechenden Bildsignale werden einer dem Operationsmikroskop zugeordneten Auswerteeinheit zugeführt, welche aus der erfassten Bildinformation die räumliche Lage der Markierung relativ zum Operationsmikroskop zu errechnen vermag.

Die US 5,999,837 offenbart ein Operationsmikroskop, bei dem Position und Lage eines Objekts im Sehfeld des Operationsmikroskops relativ zum Operationsmikroskop durch Erfassung der Lage des Operationsmikroskopes relativ zum Operationsraum und durch Erfassung der Lage des entsprechenden Objekts relativ zum Operationsraum zum Beispiel mit Hilfe eines Pointers bestimmt werden können.

Aufgabe der Erfindung ist es, ein gattungsgemäßes Operationsmikroskop bereitzustellen, mit welchem die Positions- und Lageerfassung einfacher, präziser und störungsanfälliger durchgeführt werden kann.

Diese Aufgabe wird durch die Merkmale in Anspruch 1 gelöst.

Denn durch die beiden, in Abstand zueinander angeordnete Bildsensoren des Operationsmikroskops, kann die Positions- und Lageerfassung direkt aus den von den beiden Bildsensoren erfassten Stereo-Bildpaaren durch ein zum Beispiel aus der DE 37 20 019 C2 bekanntes Auswerteverfahren erfolgen. Dadurch können Lage und Position relativ zum Operationsmikroskop mit einer geringeren Anzahl von Messungen als bisher erfasst werden.

Aufgrund des erfindungsgemäßen Operationsmikroskops ist auch die bei der Anordnung gemäß der US 5,999,837 gegebene Gefahr von Abschattungen zwischen den Bildsensoren und dem Objekt erheblich verringert.

Gegenstand des abhängigen Anspruchs ist eine vorteilhafte Ausführungsform der Erfindung.

Die Erfindung wird im folgenden anhand von Ausführungsbeispielen unter Bezugnahme auf die beigefügten Zeichnungen erläutert.

Es zeigen:
- Figur 1:: eine schematische Darstellung einer Ausführungsform der Erfindung; und
- Figur 2:: eine perspektivische Ansicht eines derartigen Operationsmikroskops.

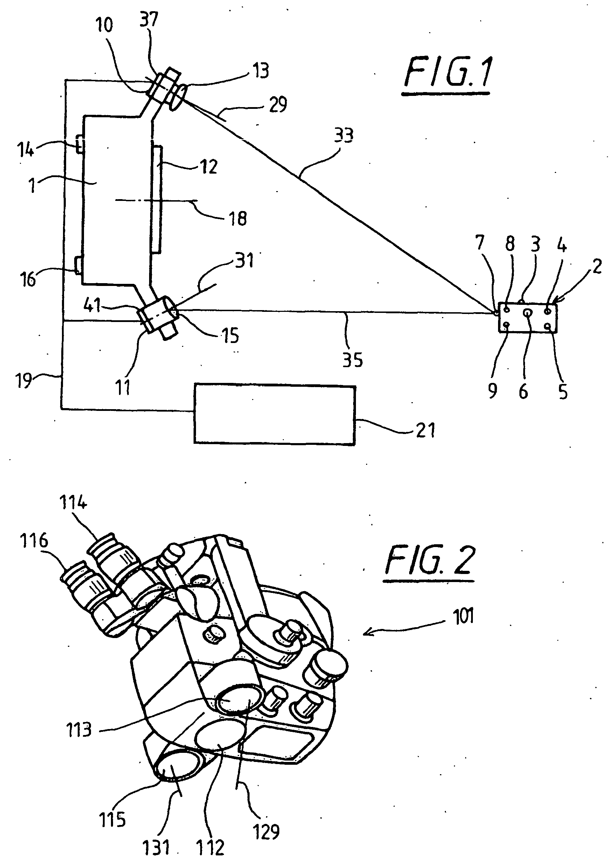

In Figur 1 ist ein erfindungsgemäßes operationsmikroskop 1 schematisch dargestellt. In dem Sehfeld des Operationsmikroskopes 1 befindet sich ein chirurgisches Instrument 2, welches als sphärische Reflektoren ausgebildete Marker 3, 4, 5, 6, 7, 8 und 9 aufweist.

Das Operationsmikroskop 1 umfaßt ein Objektiv 12, Okulare 14 und 16 sowie neben dem Objektiv 12 angeordnete Bildsensoren 10 und 11, welche zum Beispiel als CCD-Arrays ausgebildete sein können. Dabei sind die beiden Bildsensoren 10 und 11 zur Erzeugung koordinatenmäßig auswertbarer Stereo-Bildpaare in Abstand voneinander und mit unterschiedlicher Ausrichtung zueinander angeordnet.

Das Operationsmikroskop 1 umfaßt weiterhin als Kameraobjektive ausgebildete Abbildungseinrichtungen 13 bzw. 15, welche die Objekte im Sehfeld des Operationsmikroskops 1 und z. B. insbesondere die Marker 3, 4, 5, 6, 7, 8 und 9 des chirurgischen Instruments 2 auf die Bildsensoren 10 bzw. 11 abbilden. Mit 29 und 31 sind die optischen Achsen der Bildsensoren 10 bzw. 11 bezeichnet. Ferner ist mit 33 bzw. 35 der Hauptstrahl des zur Abbildung des Markers 7 auf den Bildsensor 10 bzw. 11 beitragenden Strahlenbüschels bezeichnet.

Über eine Signalleitung 19 werden die von den Bildsensoren 10 und 11 erfaßten Bilder an eine Auswerteeinheit 21 übertragen, welche aus diesen Bildern die Ortskoordinaten der Marker 3, 4, 5, 6, 7, 8 und 9 in einem durch die Anordnung der Bildsensoren 10 und 11 festgelegten und dadurch auf das Operationsmikroskop 1 bezogenen Koordinatensystems bestimmt.

Das erfindungsgemäße Operationsmikroskop kann auch mit drei oder noch mehr Bildsensoren ausgestattet sein, wobei dann lediglich statt eines Bildpaars ein Bildtrippel usw. von der Auswerteeinheit 21 auszuwerten ist.

In Figur 2 ist ein erfindungsgemäßes Operationsmikroskop in einer perspektivischen Darstellung zu erkennen. Den Elementen des Operationsmikroskops 1 der Figur 1 entsprechende Elemente tragen die gleichen Bezugszeichen vermehrt um die Zahl 100. Für ihre Beschreibung wird auf die Beschreibung zu Figur 1 verwiesen.

In Figur 2 ist insbesondere zu erkennen, daß die Abbildungseinrichtungen 113 und 115 seitlich neben dem Objektiv 112 angeordnet sind und das Objektiv 112 zwischen sich aufnehmen. Dies gewährleistet bei relativ geringem Außenabmessungen des Operationsmikroskops eine verhältnismäßig große und damit für die Bildauswertung des Stereo-Bildpaare günstige Stereobasis.

## Patentansprüche

1. Operationsmikroskop (1; 101) mit einem Objektiv (12; 112) zur Beobachtung eines Objekts und mit einer Vorrichtung zur Positions- und Lageerfassung von Objektpunkten, relativ zu dem Operationsmikroskop,
- bei dem das Operationsmikroskop (1; 101) einen ersten Bildsensor (10) und einen zweiten Bildsensor (11) umfasst, welche in Abstand voneinander angeordnet sind; wobei
- das Operationsmikroskop (1; 101) eine das Objekt auf den ersten Bildsensor (10) abbildende Abbildungseinrichtung (13; 113) und eine das Objekt auf den zweiten Bildsensor (11) abbildende Abbildungseinrichtung (15; 115) umfasst; und wobei
- eine Auswerteeinheit (21) vorgesehen ist, die aus dem Ort eines Objektpunkts in dem von dem ersten Bildsensor (10) erfassten Bild und aus seinem Ort in dem von dem zweiten Bildsensor (11) erfassten Bild sowie aus der räumlichen Anordnung der Bildsensoren (10, 11) und der Abbildungseinrichtungen (13, 15; 113, 115) Lage und Position des Objektpunkts relativ zum Operationsmikroskop (1; 101) bestimmt;
**dadurch gekennzeichnet, dass**
die Abbildungseinrichtungen (13, 113; 15, 115) seitlich neben dem Objektiv (12; 112) angeordnet sind und das Objektiv (12; 112) zwischen sich aufnehmen.

2. Operationsmikroskop (1; 101) nach Anspruch 1, **dadurch gekennzeichnet, dass** die optischen Achsen (29, 31) der das Objekt auf den ersten Bildsensor (10) abbildenden Abbildungseinrichtung (13; 113) und das Objekt auf den zweiten Bildsensor (11) abbildenden Abbildungseinrichtung (15; 115) schräg zur optischen Achse (18) des Objektives (12; 112) verlaufen.

## Claims

1. Surgical microscope (1; 101) having an objective (12; 112) for observing an object, and having an apparatus for detecting the position and attitude of object points relative to the surgical microscope,
- in which the surgical microscope (1; 101) comprises a first image sensor (10) and a second image sensor (11) which are arranged at a distance from one another;
- the surgical microscope (1; 101) comprising an imaging device (13; 113) which images the object onto the first image sensor (10), and an imaging device (15; 115) which images the object onto the second image sensor (11), and
- an evaluation unit (21) being provided which determines the position and attitude of the object point relative to the surgical microscope (1; 101) from the location of an object point in the image detected by the first image sensor (10) and from its location in the image detected by the second image sensor (11), as well as from the spatial arrangement of the image sensors (10, 11) and of the imaging devices (13, 15; 113, 115);
**characterized in that**
the imaging devices (13, 113; 15, 115) are arranged at the side next to the objective (12; 112) and hold the objective (12; 112) between them.

2. Surgical microscope (1; 101) according to Claim 1, **characterized in that** the optical axes (29, 31) of the imaging device (13; 113) which images the object onto the first image sensor (10), and of the imaging device (15; 115) which images the object onto the second image sensor (11) run obliquely to the optical axis (18) of the objective (12; 112).

## Revendications

1. Microscope chirurgical (1 ; 101) avec un objectif (12 ; 112) permettant d'observer un objet et comprenant un dispositif pour la détection de position et de situation de points d'objet, par rapport au microscope chirurgical,
- dans lequel le microscope chirurgical (1 ; 101) comprend un premier capteur d'image (10) et un deuxième capteur d'image (11) qui sont disposés à distance l'un de l'autre ; dans lequel
- le microscope chirurgical (1 ; 101) comprend un dispositif de reproduction (13 ; 113) reproduisant l'objet sur le premier capteur d'image (10) et un dispositif de reproduction (15 ; 115) reproduisant l'objet sur le deuxième capteur d'image (11) ; et dans lequel
- une unité d'analyse (21) est prévue qui détermine à partir de l'emplacement d'un point d'objet sur l'image détectée par le premier capteur d'image (10) et à partir de son emplacement sur l'image détectée par le deuxième capteur d'image (11) ainsi qu'à partir de l'agencement spatial des capteurs d'image (10, 11) et des dispositifs de reproduction (13, 15 ; 113, 115) la situation et la position du point d'objet par rapport au microscope chirurgical (1 ; 101) ;
**caractérisé en ce que**
les dispositifs de reproduction (13, 113 ; 15, 115) sont agencés latéralement à côté de l'objectif (12 ; 112) et reçoivent l'objectif (12 ; 112) entre eux.

2. Microscope chirurgical (1 ; 101) selon la revendication 1, **caractérisé en ce que** les axes optiques (29, 31) du dispositif de reproduction (13 ; 113) reproduisant l'objet sur le premier capteur d'image (10) et du dispositif de reproduction (15 ; 115) reproduisant l'objet sur le deuxième capteur d'image (11) s'étendent de façon oblique par rapport à l'axe optique (18) de l'objectif (12 ; 112).
